# EUROPEAN PATENT APPLICATION

(11) **EP 4 763 085 A1**
(43) Date of publication of application: **24.06.2026**
(21) Application number: 25224289.6
(22) Date of filing: 17.12.2025
(51) Int. Cl.: A61B 5/287, A61B 5/00, A61B 18/14

(54) **ZERO-POINT FORCE CALIBRATION FOR BASKET CATHETER**

(30) Priority: 18.12.2024 US 202418986327
(71) Applicant: Biosense Webster (Israel) Ltd., 2066717 Yokneam (IL)
(72) Inventor: GLINER, Vadim, 2066717 Yokneam (IL); FUCHS, Amit, 2066717 Yokneam (IL)
(74) Representative: Carpmaels & Ransford LLP

(57) **Abstract**

The present disclosure provides a system comprising a catheter with an expandable distal-end assembly (EDEA) having a plurality of electrodes, and a processor. The processor is configured to receive an input from a user ordering a force-zeroing measurement for the EDEA while in a patient's heart, estimate actual positions of at least some electrodes, determine canonical positions of the electrodes, compute a deviation of the actual positions from the corresponding canonical positions, and enable or prevent the force-zeroing measurement depending on the computed deviation. The system improves accuracy in force measurements by automatically determining when conditions are suitable for force-zeroing, enhancing the reliability of subsequent force measurements during medical procedures.

## Description

### FIELD OF THE INVENTION

The present invention relates generally to medical devices, and particularly to methods and systems for estimating mechanical/deformation force applied between catheter and tissue.

### BACKGROUND OF THE INVENTION

Various techniques for measuring and/or estimating the contact force exerted by a medical device inside tissue are known in the art. Effective techniques for obtaining such force measurements are constantly being improved. In particular, catheters with expandable distal-end assemblies (EDEAs) equipped with multiple electrodes are used for mapping and ablation procedures in the heart. These catheters often incorporate force sensing capabilities to measure the contact force between the catheter and the heart tissue.

Catheters with EDEAs, such as basket catheters, can expand within heart chambers to make contact with the chamber walls. The electrodes on these catheters can be used for various purposes, including sensing electrical activity in the heart, delivering ablation energy, and providing position information. The ability to accurately determine the positions of these electrodes within the heart is crucial for many cardiac procedures.

Position sensing technologies for medical devices have evolved significantly over the years. These technologies may utilize various physical principles, such as electromagnetic fields, electrical impedance measurements, or mechanical sensors, to determine the location and orientation of catheter electrodes within the body. Advanced signal processing techniques are often employed to improve the accuracy and reliability of these position measurements.

The mechanical properties of catheters, particularly those with expandable components, can be complex. Catheters are often designed to be flexible enough to navigate through blood vessels while still maintaining sufficient rigidity to apply controlled forces when necessary. The materials used in catheter construction, such as Nitinol, contribute to these mechanical characteristics.

As medical procedures become increasingly sophisticated, there is ongoing research and development aimed at improving the precision and reliability of medical devices and associated measurement techniques. This includes efforts to enhance force sensing capabilities, position tracking accuracy, and the overall performance of catheter-based systems used in cardiac procedures.

The present invention will be more fully understood from the following detailed description of the examples thereof, taken together with the drawings in which:

### BRIEF DESCRIPTION OF THE DRAWINGS

Fig. 1 is a schematic, pictorial illustration of a catheter-based position-tracking and ablation system, in accordance with an example of the present invention;
Fig. 2 is a schematic, pictorial illustration of a distal-end assembly of a catheter of the system depicted in Fig. 1, exemplifying different locations for the distal-end assembly inside a patient heart, in accordance with an example of the present invention; and
Fig. 3 is a flow chart that schematically illustrates a method for estimating a zero-point force calibration, in accordance with an example of the present invention.

### DETAILED DESCRIPTION OF EXAMPLES

### OVERVIEW

Calibration procedures play an important role in ensuring the accuracy of force measurements obtained from medical devices. These procedures may involve establishing baseline measurements or determining reference positions under controlled conditions. The specific calibration requirements can vary depending on the type of device and the intended application.

Some medical procedures, such as pulsed field ablation (PFA) and radiofrequency (RF) ablation of tissue in a patient heart, benefit from sensing contact force applied on the tissue during ablation. The distal-end assembly may comprise, for example, a basket assembly comprising ablation electrodes coupled to flexible splines.

One challenge in using such catheters, especially larger ones that may occupy a significant volume of a heart chamber, is accurately determining the zero-force state, i.e., the fully relaxed state of the distal-end assembly when not in contact with tissue. This is used for calibrating force measurements and ensuring precise force application during procedures. Traditional methods for zero-point force calibration, also referred herein as zero-point force calibration measurement often rely on visual confirmation that the catheter appears to be in the middle of a heart chamber, assuming no contact with the tissue. However, for larger catheters it may be difficult to confirm no-contact with the wall in 3D space even if the catheter seems to be in the center of the chamber. Even small deformations can result in unintended contact, making it difficult to ensure a true absence of contact force. The relatively large dimension of the Nitinol basket, coupled with the complex geometry of the heart chamber, present a significant challenge in ensuring absence of contact during a zero-point force calibration.

Furthermore, the force-deformation relationship in these catheters is often highly nonlinear, with the catheter exhibiting the most sensitivity to small forces when in its near canonical state. This sensitivity to small forces adds complexity to the task of accurately determining the zero-force state based on visual inspection. For example, the zero-state may be erroneously determined during unintended contact associated with small deformations that are not visually apparent.

An automated approach described herein helps address this complexity in certain examples by identifying when the electrodes on the EDEA are in their canonical positions and activating, enabling or preventing zero-point force calibration based on computed deviations from canonical positions. This automated assessment capability may help improve the accuracy of zero-point force calibration in some examples. Improved zeroing techniques could potentially enhance the overall accuracy of force measurements during procedures, leading to better patient outcomes and more efficient workflows.

Consider, for example, a basket catheter. Basket type catheters are typically constructed with Nitinol splines having elastic-plastic properties, or with any other material. Since Nitinol is sensitive to temperature and in-vivo conditions, the neutral, fully relaxed shape of the Nitinol basket when positioned within the void of the heart chamber may be different from its shape outside of the patient.

Therefore, it is necessary to pre-determine the shape of the Nitinol basket, when no mechanical forces are applied to it by the tissue of the heart, prior to estimating the mechanical force exerted on the Nitinol basket during the procedure. This shape is referred to herein as a "zero-point force calibration".

Additionally, the system may utilize the Touch Proximity Index (TPI), which is based on impedance, and electrocardiogram (ECG) signals to determine when the electrodes are in contact with or in proximity to the tissue. TPI provides an indication of the proximity of the electrodes to the tissue, while ECG signals are detected when the electrodes are close to or in contact with the tissue but may not be detected when the electrodes are distanced from the tissue. By integrating this information, the system can make an optimal assessment as to when to perform zero-point force calibration, thereby ensuring accurate force measurements during medical procedures.

In view of the foregoing, it would be desirable to provide a catheter-based diagnostic and/or treatment system that is able to automatically obtain a zero-point force calibration applied to a distal-end assembly of a catheter and/or guide a user as to when zeroing may be initiated.

Examples of the present invention that are described hereinbelow provide techniques for obtaining a zero-point force calibration applied, prior to an ablation or sensing procedure, between tissue in question (e.g., of a patient heart) and an expandable distal-end assembly (EDEA) of a catheter. The disclosed techniques rely on quantifying and using the elastic properties of a deformable component of the distal-end assembly.

In some examples, the system may include a catheter comprising an expandable distal-end assembly (EDEA) having a plurality of electrodes disposed thereon, and a processor. The processor may be configured to receive an input from a user ordering a zero-point force calibration for the EDEA while the EDEA is in a heart of a patient.

The processor may determine canonical positions of at least some of the electrodes, e.g., relative to the reference point. While the EDEA is in-vivo, the processor may estimate actual positions of at least some of the electrodes. The processor may then compute a deviation of the actual positions from the corresponding canonical positions. Based on the computed deviation, the processor may enable or disable (in other words, permit or override) the zero-point force calibration ordered by the user.

Provided that the zero-point force calibration is enabled, the processor may actuate the zero-point force calibration based on receiving a command from a user. The processor may then compute a force based on the deviation of the actual positions and on output from the zero-point force calibration, and display the computed force on an electronic display.

The process of enabling and disabling the zero-point force calibration can be repeated in-vivo. For example, the processor may disable the zero-point force calibration measurement based on the deviation exceeding the deviation threshold, and reinitiate another zero-point force calibration measurement, based on the deviation falling below the deviation threshold.

This automated decision-making process may be important because the system may be able to assess the suitability of conditions for zero-point force calibration more accurately than a physician in some examples.

In some examples, if the computed deviation exceeds a predetermined threshold, the processor may disable the use of the zero-point force calibration. Conversely, if the computed deviation is below the threshold, the processor may enable the use of the zero-point force calibration. The threshold may be adjustable based on factors such as the specific procedure being performed or the patient's anatomy.

Alternatively, the system may look for a minimum deviation over a sensing period. During this period, the processor continuously monitors the deviations of the actual positions from the canonical positions and identifies where the deviation is at its minimum. The measurement having this minimum deviation can then be used as the zero-point force calibration.

The system may provide feedback to the user regarding the deviation and allow the user to proceed with the zero-point force calibration in some cases. This feedback may include visual or auditory cues to guide the user in repositioning the EDEA to achieve conditions more suitable for force-zeroing.

By automating the decision to perform zero-point force calibration, the system may help ensure more accurate and reliable force measurements during medical procedures. This may lead to improved patient outcomes and more efficient workflows in some cases.

In some examples, the catheter includes an EDEA having a plurality of electrodes disposed thereon. The EDEA is constructed as a basket comprising one or more splines that can deform in response to mechanical force and resume their original shape in the absence of force. The splines are typically made of a shape-memory alloy such as Nitinol, which has elasticity as well as plastic properties and is biocompatibility.

In some examples, the electrodes on the EDEA serve multiple purposes. One electrode may function as a transmitter, while others act as receivers. The transmitter is preferably single-axis position sensor (TAS). The receiving electrodes are typically single-axis sensors (SAS) oriented in different 3D locations and orientations, designed to detect signals from the transmitter.

In some examples, the system includes position sensing capabilities for the electrodes on the EDEA. The position sensing may allow for determination of the actual positions of the electrodes within the patient's heart.

In some examples, the transmitter is positioned on the distal end of the shaft, and the receivers are positioned on the distal ends of the EDEA. This configuration allows the transmitter to emit signals that are detected by the receivers, enabling the system to accurately determine the positions of the electrodes and assess the deformation of the EDEA.

Additionally, the system may use a Bezier curve-based approach. This method may involve sensing the position and location of the catheter shaft and single-axis sensors (SAS) at the distal end of the basket. The system may then determine the shape of each spline using a Bezier curve, which in turn may be used to determine the location of each electrode on the spline.

In some examples, the sensors can be actual coils or printed coils.

In some examples, the position sensing may utilize multiple approaches. For example, the system may employ an advanced current localization (ACL) technique to determine the location of each electrode. This method may involve applying currents and measuring resulting voltages to calculate electrode positions.

The system may combine data from both the ACL and Bezier curve-based approaches to enhance the accuracy of the electrode position estimates.

In some examples, the system may use a model of the catheter mechanics to further improve the electrode position estimation based on these two methods. In this method constraints resulting from the mechanical properties of the catheter must be incorporated. These constraints may include the elasticity and deformation characteristics of the catheter materials, the expected response to applied forces, and the geometric configuration of the splines. By integrating these mechanical constraints, the system can enhance the precision of the Bezier curve representation and improve the accuracy of the electrode position estimates.

The position sensing capabilities may enable the system to track the deformation of the EDEA in real-time as it interacts with the heart tissue. This information may be used to compute the deviation of the actual electrode positions from their canonical positions, which in turn may be used to determine when conditions are suitable for force-zeroing measurements.

In some examples, the catheter comprises a transmitter and an EDEA having one or more receivers, typically implemented using electrical coils. The transmitter is coupled to a shaft or an irrigation apparatus of the catheter. The one or more receivers are coupled to respective deformable component(s) of the EDEA. For example, (i) in an EDEA comprising a basket, one or more receiver(s) may be coupled to each spline of the basket.

The one or more receivers are electrically connected to the processor and the transmitter is electrically connected to the processor or to a signal generator controlled by the processor. In some examples, the electrical connection may be carried out using (i) electrical wires or traces running between the proximal and distal ends of the catheter, e.g., between the EDEA and an operating console of the system that comprises the processor, or (ii) wirelessly, using wireless devices coupled to the proximal and distal ends of the catheter.

In some examples, the processor is configured to apply to the transmitter a transmission signal, referred to herein as a first signal, and at least one of the receivers is configured to produce a receiving signal, referred to herein as a second signal, in response to receiving the first signal.

In some examples, during the zero-point force calibration and prior to the zero-point force calibration, a physician inserts the EDEA into the patient heart, expands the EDEA to an expanded position, places the EDEA in contact with the tissue and moves the EDEA among multiple different locations inside the heart. For each location of the EDEA in the heart, the processor is configured to estimate, based on an input from a user ordering a zero-point force calibration for the EDEA while the EDEA is in a heart of a patient.

Alternatively, the system may perform the zero-point force calibration automatically without involving the user. In this method, the processor continuously monitors the deviations of the actual positions from the canonical positions as the EDEA is moved within the heart. The processor identifies where the deviation is at its minimum and automatically performs the zero-point force calibration based on this minimum deviation.

The processor may estimate actual positions of at least some of the electrodes and compare them to canonical positions of the at least some of the electrodes. The processor may then compute a deviation of the actual positions from the corresponding canonical positions. Based on the computed deviation, the processor may enable or prevent the zero-point force calibration ordered by the user.

In some examples, the processor is configured to record the output from the SAS sensors or other deformation-sensing sensors throughout the zero-point force calibration.

In some examples, if the computed deviation exceeds a predetermined threshold, the processor may prevent the recording of the zero-point force calibration and then will enable performing another zero-point force calibration. Conversely, if the computed deviation is below the threshold, the processor may enable the recording of zero-point force calibration.

In some examples, the system/processor may provide visual or auditory feedback using a display to guide the user in repositioning the EDEA to achieve conditions suitable for zero-point force calibration. This feedback may include real-time indicators of the current deviation and suggestions for catheter movements that may reduce the deviation.

The threshold may be adjustable based on factors such as the specific procedure being performed or the patient's anatomy. In some examples, the processor may provide feedback to the user regarding the deviation and allow the user to decide whether to proceed with the zero-point force calibration. In some examples, the threshold deviation will be pre-determined. The user can also adjust the threshold manually. More likely, the system would use the deviation from canonical positions, together with Touch Proximity Index (TPI) and electrocardiogram (ECG) recordings, to find the optimal moment to capture the zero state. In this case, thresholds could be used as hard stops, meaning that if the deviation exceeds this threshold, it is a clear indication that zero-point force calibration cannot be performed.

The disclosed techniques improve the force sensing capability. Force sensing may be used to improve the quality of cardiac procedures, e.g., PFA and/or RF ablation and electrophysiological mapping, by improving the accuracy and reducing the complexity of sensing mechanical force applied between ablation electrodes and tissue intended to be ablated during the RF ablation procedure. The disclosed techniques are also applicable, *mutatis mutandis,* to any other medical procedure that requires accurate sensing of mechanical force between a medical device pressed against tissue.

### SYSTEM DESCRIPTION

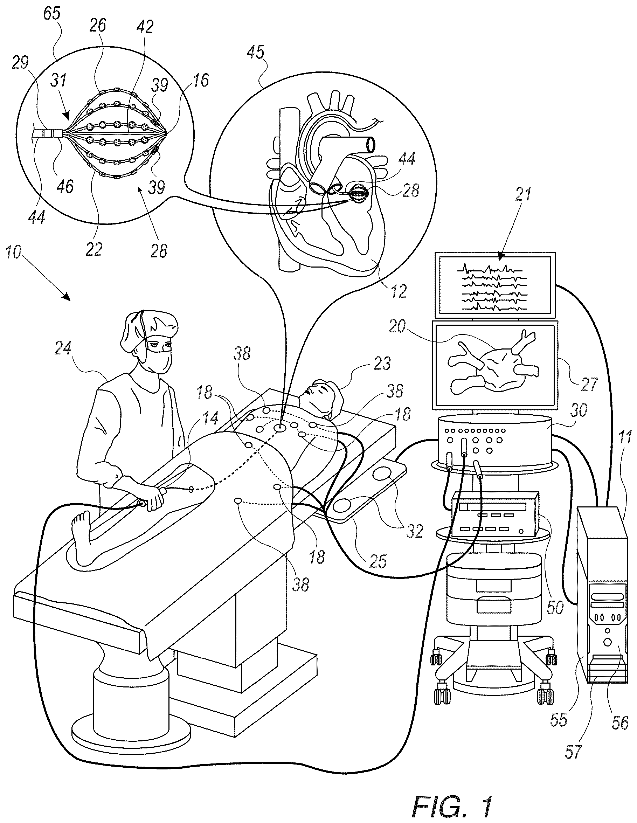

Fig. 1 is a schematic, pictorial illustration of a catheter-based position-tracking and ablation system 10, in accordance with an example of the present invention.

System 10 includes one or more catheters, which are percutaneously inserted by physician 24 through the patient's vascular system into a chamber or vascular structure of a heart 12. Typically, a delivery sheath catheter is inserted into the left or right atrium near a desired location in heart 12. Thereafter, one or more catheters can, in turn, be inserted into the delivery sheath catheter to arrive at the desired location. The one or more catheters may include catheters dedicated to sensing intracardiac electrogram (IEGM) signals, catheters dedicated to ablating and/or catheters dedicated to both sensing and ablating. An example basket catheter 14 that is configured for ablation sensing IEGM is illustrated herein. As another example, catheter 14 can be a tip catheter for ablation and sensing fitted with a contact force mechanism described in U.S. Patent 8,535,308, which is assigned to the assignee of the current application.

As seen in inset 45, physician 24 brings a basket assembly 28 (also called hereinafter "expandable distal-end assembly 28") fitted on a shaft 44 of catheter 14 into contact with the heart wall for sensing a target site in heart 12. For ablation, physician 24 similarly brings a distal end of an ablation catheter to a target site for ablating.

As seen in inset 65, basket catheter 14 is an exemplary catheter that includes one and preferably multiple electrodes 26 optionally distributed over a plurality of splines 22 at expandable distal-end assembly 28 and configured to ablate and/or sense IEGM signals. Catheter 14 additionally includes (i) a proximal position sensor 29 e.g., dual axial sensor (DAS) 29 with two orthogonal EMCs or tri-axial sensor (TAS) comprising three orthogonal EMCs embedded in a distal end 46 of shaft 44 near basket assembly 28, and (ii) three distal position sensors 39, e.g., single axial sensor (SAS) 39 comprising a single EMC for a tracking position of the distal end of basket assembly 28. Optionally and preferably, position sensors 29 and 39 are magnetic-based position sensors that include magnetic coils for sensing three-dimensional (3D) position. An example reference electrode 31 located at the base of assembly 28 is also seen.

Reference is now made back to the general view of Fig. 1. In some examples, during the navigation of distal-end assembly 28 in heart 12, a processor 56 receives signals from magnetic position sensors 29 and 39.

The method of position sensing using external magnetic fields is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

Magnetic position sensors (29, 39) (i.e., an assembly of coils) may also be operated together with an external location pad 25 that includes a plurality of magnetic coils 32 configured to generate magnetic fields in a predefined working volume. To prevent confusion among signals, the frequencies of these fields are different from any given frequency used in the local transmitter-receiver mode for contact force detection. Real-time orientation of basket assembly 28 of catheter 14 can, in this way, be calculated from tracked locations of sensors 29 and 39 (locations being tracked using magnetic fields generated with location pad 25 and sensed by magnetic-based position sensors 29 and 39). This relative orientation is manifested by an angle formed between a distal end 46 and a longitudinal axis 42 of expandable assembly 28 (to a distal edge 16 of the assembly).

Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25, as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses that may be used to effect irreversible electroporation (IRE), or combinations thereof.

Patient interface unit (PIU) 30 is configured to establish electrical communication between catheters, electrophysiological equipment, power supply, and a workstation 55 for controlling operation of system 10. Electrophysiological equipment of system 10 may include, for example, multiple catheters, location pad 25, body surface ECG electrodes 18, electrode patches 38, ablation energy generator 50, and recorder 11. Optionally and preferably, PIU 30 additionally includes processing capability for implementing real-time computations of catheter locations and for performing ECG calculations.

Workstation 55 includes memory 57, processor unit 56 with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered anatomical map 20, (iii) displaying real-time location and orientation of multiple catheters within the heart chamber, and (iv) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

While Fig. 1 describes a basket assembly, the figure and disclosed method are applicable, *mutatis mutandis,* to a multi-ray assembly and a balloon assembly.

This particular configuration of system 10 is shown by way of example, in order to illustrate certain problems that are addressed by examples of the present invention and to demonstrate the application of these examples in enhancing the performance of such a system. Examples of the present invention, however, are by no means limited to this specific sort of example system, and the principles described herein may similarly be applied to other sorts of medical systems and procedures.

### DISTAL-END ASSEMBLY AND FORCE-ZEROING MEASUREMENT

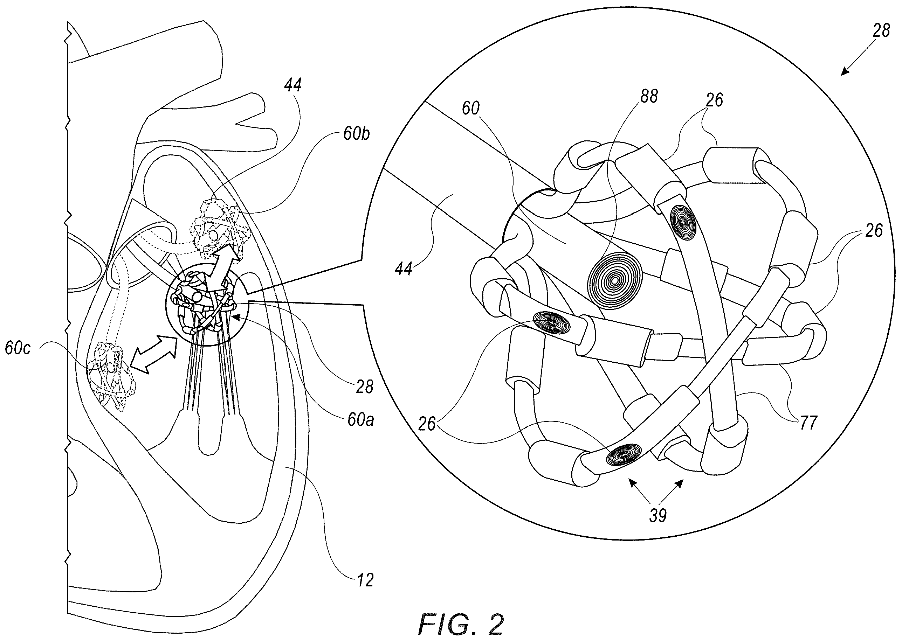

Fig. 2 is a schematic, pictorial illustration of expandable distal-end assembly (EDEA) 28 in an expanded position in three exemplified locations 60A, 60B, and 60C inside heart 12, in accordance with an example of the present invention.

When distal-end assembly 28 is located within center 60A of heart 12, no mechanical force is exerted by heart 12 tissue 47 on distal-end assembly 28 and therefore neutral shape is preserved, suitable for zero-point force calibration.

When distal-end assembly 28 is located near and in contact with tissue at location 60B of heart 12, some small mechanical force is exerted on distal-end assembly 28 by the tissue 47. However, the mechanical force is lower than a certain threshold, of about 1 gram, required to deform the neutral shape of distal-end assembly 28.

Lastly, when distal-end assembly 28 is located in contact with tissue 47 at location 60C of heart 12, a non-negligible mechanical force is exerted on distal-end assembly 28. The mechanical force is higher than a certain threshold required to deform the neutral shape of distal-end assembly 28.

As can be appreciated, from among the three locations 60A, 60B and 60C, the measurement taken at location 60A is the most suitable for serving as a zero-point force calibration measurement. In some examples, processor 56 identifies this measurement and saves it for later use.

An inset on the right-hand side of Fig. 2 shows the structure of distal-end assembly 28 in greater detail. In this example, distal-end assembly 28 is a basket assembly that comprises multiple splines 22.

In some examples, one or more ablation electrodes 26 are coupled to each spline 22 and are configured to apply ablation pulse(s) to tissue of heart 12. The ablation pulse(s) are intended to kill cells of the tissue in question and to produce, instead of the tissue, a lesion that prevents or reduces the propagation of electrophysiological (EP) waves through the ablated tissue.

In some examples, the one or more ablation electrodes 26 are also used to sense impedance for the TPI and to sense ECG signals for mapping purposes. This dual functionality of the electrodes enhances the system's ability to accurately determine the proximity and contact state of the electrodes with the tissue, thereby improving the precision of both ablation and mapping procedures.

In some examples, distal-end assembly 28 comprises a plurality of electrodes (26), including a transmitter 88 and one or more receivers 99. Transmitter 88 is coupled to a rigid component of distal-end assembly 28. In other examples, transmitter 88 may be coupled to any other suitable rigid component of distal-end assembly 28, such as shaft 44. In the context of the present disclosure and in the claims, the term "rigid" refers to a component of catheter 14 that moves together with the catheter and whose location is not affected by any force, such as contact force, applied to distal-end assembly 28, as will be described in detail below. In other words, transmitter 88 has the same angular velocity and the same angular acceleration of the distal end of shaft 44.

In some examples, one or more receivers 99 are coupled to respective elastic components of distal-end assembly 28. In the present example, one receiver 99 is coupled to each spline 22 of the basket. Note that in the example of Fig. 2, only three receivers 99 are shown, but a receiver 99 is coupled to each spline 22 even though some of receivers 99 are hidden by the isometric perspective of distal-end assembly 28, and therefore, are not shown in the example configuration of Fig. 2.

In some examples, transmitter 88 and receivers 99 may be implemented using electrical coils that are coupled to splines 22, respectively. The coils of transmitter 88 and receivers 99 are electrically connected to processor 56, using any suitable connection techniques.

In some examples, receivers 99 are electrically connected (e.g., via traces of the aforementioned flexible PCB, and via catheter 14) to processor 56. Moreover, transmitter 88 is electrically connected to processor 56 (e.g., via wires running between (i) the distal end of shaft 44 or irrigator 60, and (ii) the proximal end of catheter 14). Additionally, or alternatively, transmitter 88 may be electrically connected to a pulse generator (not shown) controlled by processor 56 and configured to apply one or more pulses using transmitter 88 as will be described below.

In other examples, the electrical connection between (i) transmitter 88 and/or receivers 99 and (ii) processor 56, may be carried out using wireless devices (not shown) coupled to the proximal and distal ends of catheter 14.

In some examples, processor 56 is configured to control transmitter 88 to apply a transmission signal, also referred to herein as a first signal. In response to receiving the first signal, at least one of and typically all receivers 99, are configured to produce a receiving signal, also referred to herein as a second signal.

In some examples, the second signal produced by a given receiver 99 is indicative of the distance between transmitter 88 and given receiver 99, as will be described below.

In some examples, during the zero-point force calibration procedure and prior to the ablation procedure, physician 24 inserts distal-end assembly 28 into a cavity of heart 12. Subsequently, physician 24 expands distal-end assembly 28 to an expanded position (as shown in the example of Fig. 2), presses a zeroing button, and maneuvers distal-end assembly 28 within heart 12 to perform the zero-point force calibration procedure.

In some examples, during the zero-point force calibration procedure, physician 24 moves EDEA 28 to multiple locations within heart 12, e.g., locations 60A, 60B and 60C. As noted above, the zero-point force calibration measurement taken at location 60A is the measurement that is best suited to serve as a zero-point force calibration measurement.

In some examples, at the end of the zero-point force calibration procedure, physician 24 presses the zeroing button to mark the end of the zero-point force calibration procedure.

In some examples, during the zero-point force calibration procedure, the processor 56 is configured to control transmitter 88 to apply the transmission signal, until the physician 24 ends the zeroing procedure.

In some examples, processor 56 is configured to estimate the mechanical force applied to the spline with a typical sensitivity of about 5 grams also referred to herein as gram-force (GF), whereas a typical value of mechanical force applied in such procedures is between about 0 grams and 100 grams.

Thus, the disclosed techniques improve the quality of ablation procedures by improving the accuracy of the estimated mechanical force applied between ablation electrodes 26 and the tissue of heart 12, which is intended to be ablated during the RF ablation procedure described above. The disclosed techniques are also applicable, *mutatis mutandis,* to any other medical procedure that requires accurate and stable sensing of mechanical force between a medical device pressed against tissue.

The configuration of distal-end assembly 28 is provided in Fig. 2 by way of example, in order to illustrate certain problems that are addressed by examples of the present invention and to demonstrate the application of these examples in enhancing the performance of such a distal end for treating arrhythmias in patient heart. Examples of the present invention, however, are by no means limited to this specific sort of example distal-end assembly, and the principles described herein may similarly be applied to other sorts of catheters used in any suitable sort of medical systems and procedures that require measurement or estimation of mechanical force applied to any suitable type of medical device pressed against any suitable tissue of a patient.

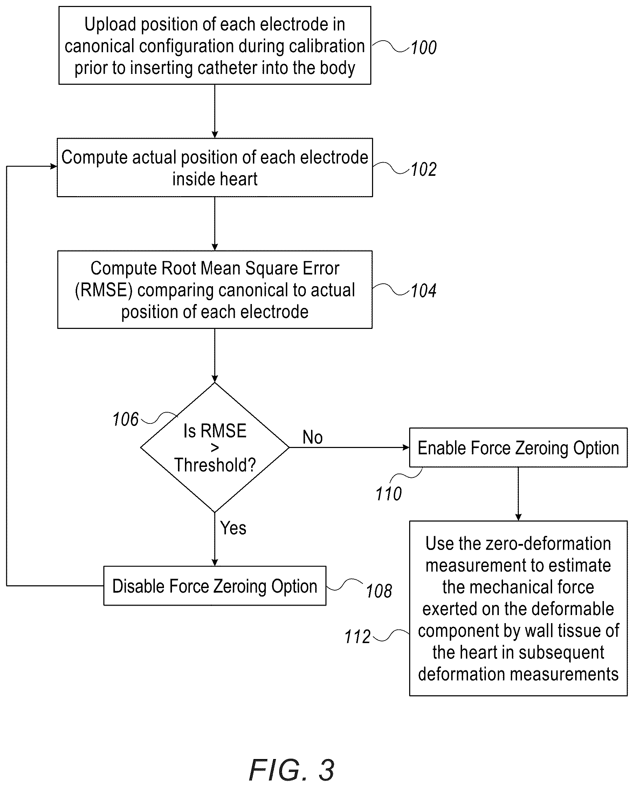

Fig. 3 is a flow chart that schematically illustrates a method for estimating a zero-point force calibration measurement for distal-end assembly 28 inside tissue of heart 12, in accordance with an example of the present invention.

The method begins at catheter pre-insertion step 100, wherein the position of each electrode in canonical configuration is uploaded to processor 56 during calibration prior to inserting catheter into the body.

At a sensing position step 102, processor 56 computes the actual position of each electrode inside the heart.

Following this, at a compute position error step 104, processor 56 computes the Root Mean Square Error (RMSE) between the preloaded canonical positions and the respective actual positions of the electrodes.

In an optimization step (not shown in the flow-chart), the system is configured to optimize the zero-point force calibration measurement by identifying the best zeroing time within the threshold. This optimization is based on a cost function that considers multiple factors, including the minimum RMSE, minimum TPI, and minimum ECG gain.

At a decision point step 106, processor 56 compares the computed RMSE to a predetermined threshold value.

If the RMSE is greater than the threshold (Yes branch), the process moves to a zero-point force calibration option disabling step 108.

In step 108, any zero-point force calibration measurements ordered by the user are disabled. The method loops back to repeat on steps 102-106.

If the RMSE is not greater than the threshold (No branch), the process moves to a zero-point force calibration option enabling step 110, where the zero-point force calibration option is enabled.

In step 110, the zero-point force calibration measurement is recorded and used in a following deformation measurements step 112 to estimate the mechanical force exerted on the deformable component by wall tissue of the heart in subsequent deformation measurements, e.g., during an ablation procedure.

The flow of Fig. 3 is an example flow that is depicted purely for the sake of conceptual clarity. Any other suitable flow can be used in alternative examples. For example, in some examples, the processor monitors an impedance and/or an intracardiac electrogram (ICEG) at each of the plurality of electrodes, and enables or disables the zero-point force calibration measurement based on pre-defined criteria defined for the impedance and/or ICEG, as well as based on comparing of the deviation to the deviation threshold.

In some examples the deviation threshold is dynamically updated based on the deviation computed over a defined time period. In some examples the deviation threshold is user defined.

In one example, the command to initiate zero-point force calibration is based on user input requesting the zero-point force calibration. In another example, the command is automated based on the processor identifying a pre-defined event.

In an example, the processor renders a virtual representation of the EDEA, and updates the shape of the virtual representation based on the monitored actual positions of the plurality of electrodes.

In another example, the EDEA is in the form of a basket including a plurality of deformable splines, and the actual positions of the plurality of electrodes are determined based on a mechanical model of the EDEA and output from a plurality of magnetic based position sensors mounted on the deformable splines. Typically, the canonical configuration of the EDEA is predetermined ex-vivo during a calibration procedure.

Although the examples described herein mainly address estimation of force-zeroing measurement for an expandable distal-end assembly of an RF ablation catheter and tissue intended to be ablated, the methods and systems described herein can also be used in other applications, such as in any application that requires accurate measurement of contact force applied between any suitable medical device and any suitable tissue of an organ of a patient. For example, in organs of an ear-nose-throat (ENT) system of a patient.

### EXAMPLES

### Example 1:

A system includes a catheter, a processor and an electronic display. The catheter includes an expandable distal-end assembly (EDEA) including a plurality of electrodes disposed thereon. The processor is configured to store, in a memory, canonical positions of the plurality of electrodes of the catheter in relation to a reference point, to monitor actual positions of the plurality of electrodes while the EDEA is in-vivo, and to enable or disable zero-point force calibration based on comparing a deviation of the actual positions from the canonical positions to a deviation threshold. The processor is further configured to actuate the zero-point force calibration based on receiving a command, provided that the zero-point force calibration is enabled, and to compute a force based on the deviation of the actual positions and output from the zero-point force calibration. The electronic display configured is configured to display the computed force.

### Example 2:

The system according to example 1, wherein the processor is configured to disable the zero-point force calibration measurement based on the deviation exceeding the deviation threshold, and reinitiate another zero-point force calibration measurement based on the deviation falling below the deviation threshold.

### Example 3:

The system according to example 1, wherein the processor is configured to monitor one or both of impedance and intracardiac electrogram (ICEG) at each of the plurality of electrodes, and to enable or disable the zero-point force calibration measurement based on pre-defined criteria defined for one or both of impedance and ICEG as well as based on the comparing of the deviation to the deviation threshold.

### Example 4:

The system according to example 1, wherein the deviation threshold is dynamically updated based on the deviation computed over a defined time period.

### Example 5:

The system according to example 1, wherein the deviation threshold is user defined.

### Example 6:

The system according to example 1, wherein the command is based on user input requesting the zero-point force calibration.

### Example 7:

The system according to example 1, wherein the command is automated based on the processor identifying a pre-defined event.

### Example 8:

The system according to example 1, wherein the processor is configured to render a virtual representation of the EDEA, and to update a shape of the virtual representation based on the actual positions of the plurality of electrodes monitored.

### Example 9:

The system according to example 1, wherein the EDEA is in the form of a basket including a plurality of deformable splines and wherein the actual positions of the plurality of electrodes are determined based on a mechanical model of the EDEA and output from a plurality of magnetic based position sensors mounted on the deformable splines.

### Example 10:

The system according to example 1, wherein the canonical configuration of the EDEA is predetermined ex-vivo during a calibration procedure.

### Example 11:

A method includes storing, in a memory, canonical positions of a plurality of electrodes, which are disposed on an expandable distal-end assembly (EDEA) of a catheter, in relation to a reference point. Actual positions of the plurality of electrodes are monitored while the EDEA is in-vivo. Zero-point force calibration is enabled or disabled based on comparing a deviation of the actual positions from the canonical positions to a deviation threshold. Provided that the zero-point force calibration is enabled, the zero-point force calibration is actuated based on receiving a command. A force is computed based on the deviation of the actual positions and output from the zero-point force calibration. The computed force is displayed.

### Example 12:

The method according to example 11, wherein enabling or disabling the zero-point force calibration comprises disabling the zero-point force calibration measurement based on the deviation exceeding the deviation threshold, and reinitiating another zero-point force calibration measurement based on the deviation falling below the deviation threshold.

### Example 13:

The method according to example 11, wherein enabling or disabling the zero-point force calibration comprises monitoring one or both of impedance and intracardiac electrogram (ICEG) at each of the plurality of electrodes, and enabling or disabling the zero-point force calibration measurement based on pre-defined criteria defined for one or both of impedance and ICEG as well as based on the comparing of the deviation to the deviation threshold.

### Example 14:

The method according to example 11, and comprising dynamically updating the deviation threshold based on the deviation computed over a defined time period.

### Example 15:

The method according to example 11, wherein the deviation threshold is user defined.

### Example 16:

The method according to example 11, wherein the command is based on user input requesting the zero-point force calibration.

### Example 17:

The method according to example 11, wherein the command is automated based identifying a pre-defined event.

### Example 18:

The method according to example 11, and comprising rendering a virtual representation of the EDEA, and updating a shape of the virtual representation based on the actual positions of the plurality of electrodes monitored.

### Example 19:

The method according to example 11, wherein the EDEA is in the form of a basket including a plurality of deformable splines, and wherein determining the actual positions of the plurality of electrodes is based on a mechanical model of the EDEA and output from a plurality of magnetic based position sensors mounted on the deformable splines.

### Example 20:

The method according to example 11, wherein the canonical configuration of the EDEA is predetermined ex-vivo during a calibration procedure.

It will thus be appreciated that the examples described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A system (10), comprising:
a catheter (14), comprising an expandable distal-end assembly - EDEA - (28) including a plurality of electrodes (26) disposed thereon; and
a processor (56), which is configured to:
store, in a memory (57), canonical positions of the plurality of electrodes of the catheter in relation to a reference point;
monitor actual positions of the plurality of electrodes while the EDEA is in-vivo;
enable or disable zero-point force calibration based on comparing a deviation of the actual positions from the canonical positions to a deviation threshold;
provided that the zero-point force calibration is enabled, actuate the zero-point force calibration based on receiving a command;
compute a force based on the deviation of the actual positions and output from the zero-point force calibration; and
an electronic display (27) configured to display the computed force.

2. The system according to claim 1, wherein the processor is configured to disable the zero-point force calibration measurement based on the deviation exceeding the deviation threshold, and reinitiate another zero-point force calibration measurement based on the deviation falling below the deviation threshold.

3. The system according to claim 1, wherein the processor is configured to monitor one or both of impedance and intracardiac electrogram (ICEG) at each of the plurality of electrodes, and to enable or disable the zero-point force calibration measurement based on pre-defined criteria defined for one or both of impedance and ICEG as well as based on the comparing of the deviation to the deviation threshold.

4. The system according to any of claims 1-3, wherein the deviation threshold is dynamically updated based on the deviation computed over a defined time period.

5. The system according to any of claims 1-3, wherein the deviation threshold is user defined.

6. The system according to any of claims 1-3, wherein the command is based on user input requesting the zero-point force calibration.

7. The system according to any of claims 1-3, wherein the command is automated based on the processor identifying a pre-defined event.

8. The system according to any of claims 1-3, wherein the processor is configured to render a virtual representation of the EDEA, and to update a shape of the virtual representation based on the actual positions of the plurality of electrodes monitored.

9. The system according to any of claims 1-3, wherein the EDEA is in the form of a basket including a plurality of deformable splines and wherein the actual positions of the plurality of electrodes are determined based on a mechanical model of the EDEA and output from a plurality of magnetic based position sensors mounted on the deformable splines.

10. The system according to any of claims 1-3, wherein the canonical configuration of the EDEA is predetermined ex-vivo during a calibration procedure.

11. A method, comprising:
storing, in a memory (57), canonical positions of a plurality of electrodes (26), which are disposed on an expandable distal-end assembly - EDEA - (28) of a catheter (14), in relation to a reference point;
monitoring actual positions of the plurality of electrodes while the EDEA is in-vivo;
enabling or disabling zero-point force calibration based on comparing a deviation of the actual positions from the canonical positions to a deviation threshold;
provided that the zero-point force calibration is enabled, actuating the zero-point force calibration based on receiving a command;
computing a force based on the deviation of the actual positions and output from the zero-point force calibration; and
displaying the computed force.

12. The method according to claim 11, wherein enabling or disabling the zero-point force calibration comprises disabling the zero-point force calibration measurement based on the deviation exceeding the deviation threshold, and reinitiating another zero-point force calibration measurement based on the deviation falling below the deviation threshold.

13. The method according to claim 11, wherein enabling or disabling the zero-point force calibration comprises monitoring one or both of impedance and intracardiac electrogram (ICEG) at each of the plurality of electrodes, and enabling or disabling the zero-point force calibration measurement based on pre-defined criteria defined for one or both of impedance and ICEG as well as based on the comparing of the deviation to the deviation threshold.

14. The method according to any of claims 11-13, wherein the EDEA is in the form of a basket including a plurality of deformable splines, and wherein determining the actual positions of the plurality of electrodes is based on a mechanical model of the EDEA and output from a plurality of magnetic based position sensors mounted on the deformable splines.

15. The method according to any of claims 11-13, wherein the canonical configuration of the EDEA is predetermined ex-vivo during a calibration procedure.
